# FASCICULE DE BREVET EUROPEEN

(11) **EP 3 807 902 B1**
(45) Date de publication et mention de la délivrance du brevet: **10.08.2022**
(21) Numéro de dépôt: 19753194.0
(22) Date de dépôt: 13.06.2019
(51) Int. Cl.: G16H 40/60, A61B 17/44, A61B 17/00, A61B 34/20

(54) **INSTRUMENT OBSTÉTRICAL NOTAMMENT DE TYPE FORCEPS POUR L'EXTRACTION D'UN FOETUS AINSI QU'UN DISPOSITIF D'ASSISTANCE À L'EXTRACTION ÉQUIPÉ D'UN FORCEPS TEL QUE PRÉCITÉ**
GEBURTSHILFLICHES INSTRUMENT, INSBESONDERE VOM ZANGENTYP, ZUR EXTRAKTION EINES FÖTUS, SOWIE ASSISTENZVORRICHTUNG ZUR EXTRAKTION, AUSGESTATTET MIT EINER ZANGE WIE OBEN ERWÄHNT
OBSTETRICAL INSTRUMENT, IN PARTICULAR OF THE FORCEPS TYPE, FOR EXTRACTING A FETUS, AS WELL AS AN ASSISTANCE DEVICE FOR EXTRACTION EQUIPPED WITH A FORCEPS AS MENTIONED ABOVE

(30) Priorité: 15.06.2018 FR 1870712
(43) Date de publication de la demande: 21.04.2021
(73) Titulaire: CHRU de Lille, 59800 Lille (FR)
(72) Inventeur: RUBOD, Chrystèle, 59800 Lille (FR); COSSON, Michel, 59800 Lille (FR); JEAN, Estelle, 59800 Lille (FR); GAULTIER, Franck, 59800 Lille (FR); BRIEU, Mathias, 59800 Lille (FR)
(74) Mandataire: Ipside
(86) Numéro de dépôt international: PCT/IB2019/054939
(87) Numéro de publication internationale: WO 2019/239362

(56) Documents cités:
- WO-A1-2010/043860
- WO-A2-2004/108010
- US-A- 3 785 381
- US-B2- 9 554 828

## Description

### Domaine technique

L'invention se situe dans le domaine technique de la chirurgie obstétricale et plus particulièrement dans l'aide à l'accouchement à partir d'un instrument obstétrical de type forceps.

Bien que particulièrement prévu sous la forme d'un forceps, l'instrument obstétrical pourra également revêtir la forme d'autres types de pinces de préhension permettant une extraction du fœtus par des opérations de traction au niveau de la tête fœtale et par exemple sous forme de spatule.

### Technique antérieure

On connait à ce jour de nombreux types de forceps ou de spatules, ces instruments, sous leurs formes actuelles, ont principalement été développés et améliorés en fin de XIXe siècle et permettent de faciliter l'extraction du fœtus par voie basse. Les extrémités de ces instruments (notamment cuillères céphaliques) permettent d'assurer la prise sur le fœtus et ont des formes adaptées pour limiter le risque de blessures à la fois du fœtus et de la mère. Toutefois aucun instrument obstétrique ne permet d'évaluer en temps réel la force exercée sur la tête du fœtus de sorte que, notamment compte tenu des contraintes de temps ou d'espace pour le bon positionnement du forceps, il est fréquent que le forceps exerce une pression trop importante sur le fœtus. Un serrage trop important des cuillères peut entraîner des lésions osseuses du crane notamment des fractures osseuses au niveau du pariétal ou du frontal. Il peut également entraîner une paralysie des nerfs crâniens et des atteintes de l'œil. Pour le praticien, le seul moyen de contrôle de la pression est son évaluation propre qui dépend essentiellement de nombreux entraînements sur mannequin et de son expérience d'accouchements antérieurs.

Pour limiter le risque de lésion, on recourt de plus en plus à des césariennes, toutefois l'usage du forceps reste dans de nombreux cas préférable d'autant que les césariennes peuvent induire d'autres types de complications notamment chez la mère. La présente invention propose un instrument obstétrical qui constitue une amélioration par rapport aux forceps et spatules connus de l'état de la technique.

WO 2004/108010 A2 concerne un forceps obstétrical avec une poignée à détection de traction, contenant un matériel électronique dont le but est de mesurer la force de traction (et non la pression) utilisée pendant un accouchement, afin d'alerter le médecin lorsque cette force dépasse les limites de sécurité. Dans ce document il n'y a pas de moyens de mesure de position ni de pression.

US 9,554,828 B2 concerne un forceps qui comprend un ou plusieurs capteurs de position pour surveiller la position du dispositif d'accouchement et qui peut également aider à surveiller la progression du travail. Ces moyens de mesure de la position ne sont pas détachables. Le dispositif de D2 ne mesure pas la pression.

WO 2010/043860 A1 concerne un un système de forceps pour la mesure des forces de compression et de traction, y compris la mesure des forces de compression et de traction exercées sur une tête de foetus pendant un accouchement instrumental. Il n'y a pas des moyens de mesure de la position dans le dispositif de D3. Les moyens de mesure ne sont pas détachables.

US 3,785,381 concerne une forceps qui permet à l'utilisateur d'observer facilement la pression appliquée sur la tête du fœtus comprenant des moyens de pression fixés à au moins une des parties d'engagement du fœtus d'une lame (cuillère). ces moyens peuvent être détachées. Cependant, les moyens sont analogiques (un élastomère rempli d'un liquide incompressible).

### Résumé de l'invention

La présente invention concerne un instrument obstétrical notamment de type forceps pour l'extraction par traction d'un fœtus lors de l'accouchement comportant deux branches, chaque branche étant équipée à une extrémité d'un manche de préhension et à l'autre extrémité de cuillères céphaliques et tel que, selon l'invention, l'instrument comprend un module de mesure par cuillère et des moyens de liaison entre le module de mesure et la cuillère, ledit module de mesure comportant :
- des premiers moyens de mesure de la pression aptes à mesurer la pression exercée par ladite cuillère sur le fœtus,
- des seconds moyens de mesure du positionnement dans l'espace de l'instrument obstétrical,
- des moyens d'alimentation électrique,
- des moyens de transmission des mesures vers une unité de traitement.

La présente invention vise également à protéger un dispositif d'assistance comportant un instrument obstétrical tel que précité et comportant en outre une unité de traitement avec des premiers moyens de traitement pour le calcul de la pression exercée et la comparaison avec au moins un seuil de pression maximale et/ou un seuil de pression maximale par unité de temps.

### Avantages apportés

Un premier but de la présente invention est de résoudre tout ou partie des problèmes techniques liés à l'art antérieur précité.

Un autre but de la présente invention est de proposer un instrument obstétrical permettant de suivre en continu la pression exercée sur le fœtus et le trajet du forceps.

Un autre but de la présente invention est de proposer un instrument obstétrical comportant des modules de mesure indépendants du forceps, et notamment pouvant être ajoutés et retirés manuellement.

Un autre but de la présente invention est de proposer des modules de mesures adaptables sur une grande variété de forceps ou pinces de préhension ou spatules.

Un autre but de la présente invention est de proposer des modules de mesures étanches et lavables.

Un autre but de la présente invention est de proposer un dispositif d'assistance permettant de signaler au praticien des dépassements de seuils de pression ou des variations importantes dans le trajet d'extraction du fœtus par rapport à des schémas types d'extraction.

Un autre but de la présente invention est de proposer un dispositif d'assistance permettant de mesurer le déplacement de l'instrument après chaque traction.

Un autre but de la présente invention est de proposer un dispositif d'assistance permettant d'enregistrer les contractions et le rythme de contraction en temps réel.

Un autre but de la présente invention est de proposer un dispositif d'assistance permettant d'enregistrer les paramètres d'extraction pour autoriser une analyse ultérieure de l'opération d'accouchement.

### Brève description des dessins

La présente invention sera mieux comprise à la lecture d'un exemple détaillé de réalisation en référence aux figures annexées, fournies à titre d'exemple non limitatif, parmi lesquels :
La figure 1 représente un exemple de réalisation schématique d'un instrument obstétrical conforme à l'invention,
Les figures 2a et 2b représentent en vue schématique et en perspective d'un module de mesure conforme à l'invention, respectivement face externe et face interne,
La figure 3 représente en vue schématique et en perspective d'un dispositif

### Définitions

Le terme « instrument obstétrical » définit, au sens de la présente invention un forceps notamment à branches croisées tels qu'un forceps de Tarnier ou de Pajot, à branches convergentes tel qu'un forceps de Suzor ou des spatules tels que des spatules de Thierry. Le terme définit en outre tout instrument permettant l'extraction d'un fœtus et présentant une zone destinée à être appliquée en pression au niveau de la tête du fœtus et comprend notamment différents types de pince de préhension

### Description des modes de réalisation

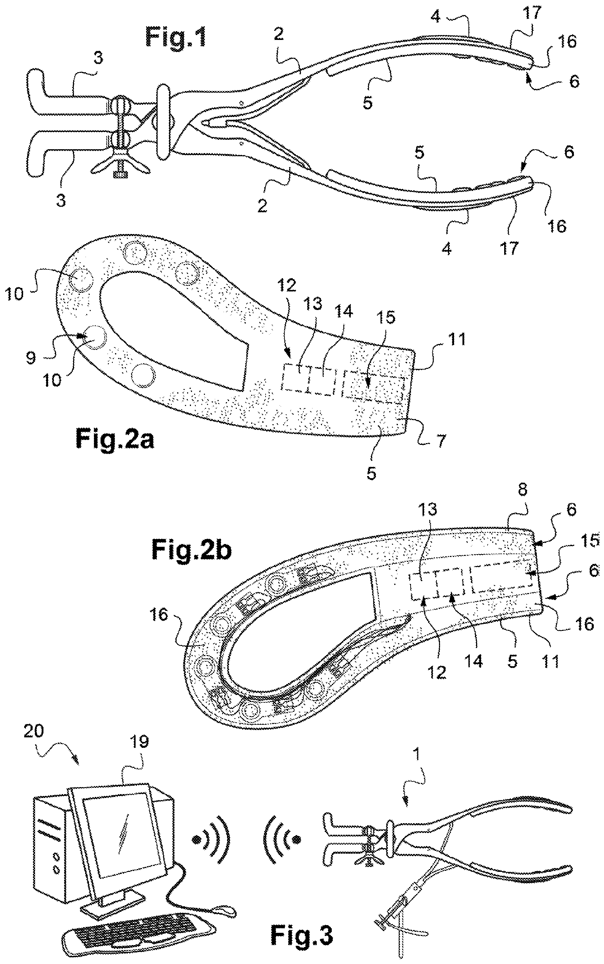

La présente invention vise à protéger un instrument obstétrical 1 tel que représenté à la figure 1.

Cet instrument obstétrical 1 comporte deux branches 2, chaque branche 2 étant équipée à une extrémité d'un manche de préhension 3 et à l'autre extrémité de cuillères céphaliques 4.

Selon l'invention, l'instrument 1, réalisé dans l'exemple des figures 1 à 3 sous la forme d'un forceps, comprend en outre un module de mesure 5 pour chaque cuillère 4 et des moyens de liaison 6 entre le module de mesure 5 et la cuillère 4.

En se reportant aux figures 2a et 2b, représentant respectivement les faces externe 7 et interne 8 du module de mesure 5 on voit que ledit module de mesure 5 comportant des premiers moyens de mesure 9 de la pression, aptes à mesurer la pression exercée par la cuillère 4 sur le fœtus.

Dans le mode de réalisation des figures annexés ces premiers moyens de mesure 9 comportent plusieurs capteurs ponctuels 10 repartis sur la face externe 7 de chaque module de mesure 5. Ces capteurs 10 pourront notamment être réalisés à partir de composants piézo-résistifs. Ces capteurs 10 sont encapsulés dans l'enveloppe 11 entourant le module de mesure 5. De manière à permettre une bonne mesure de la pression, les capteurs sont disposés de manière légèrement surélevée par rapport au plan moyen de la face externe 7.

Avantageusement la surélévation sera comprise entre 0.1 et 1 mm et l'enveloppe sera constituée en un matériau souple de sorte que lors du positionnement de l'instrument 1, il y a nécessairement un contact entre les capteurs et le foetus et en même temps un léger écrasement des capteurs 10 de manière à ce que la pression exercée par l'instrument 1 soit repartie sur la surface de la face externe 7 et non uniquement sur les capteurs 10.

Selon un autre mode de réalisation, les premiers moyens de mesure 9 de la pression comportent un seul capteur 10. Ce capteur 10 est avantageusement reparti sur au moins 50% de la surface de la rainure, de préférence de type piézo-résistifs.

Chaque module de mesure 5 comporte en outre des seconds moyens de mesure 12 du positionnement dans l'espace de l'instrument obstétrical 1.

Ces seconds moyens de mesure 12 sont de préférence réalisés par une centrale inertielle 13. Dans un mode de réalisation avantageux chaque module de mesure 5 d'une paire de modules 5 équipant un instrument 1 comprend une centrale inertielle 13. Cette disposition permet d'assurer une plus grande précision du déplacement de l'instrument 1 en réduisant l'importance de la dérive éventuelle au niveau d'une centrale inertielle 13. Toutefois, dans un autre mode de réalisation, on peut également ne prévoir, en fonction de la précision de la centrale inertielle 13 et de la précision requise pour la mesure, qu'une seule centrale inertielle 13 pour la paire de modules de mesure 5.

Les modules de mesure 5 comportent en outre des moyens d'alimentation électrique 14 et des moyens de transmission des mesures 15 vers une unité de traitement. Ces moyens d'alimentation 14 seront réalisés classiquement à partir de piles ou de batteries. Les moyens de transmission des mesures 15 comprendront de manière avantageuse un émetteur de type Bluetooth.

En se reportant plus particulièrement à la figure 2b on voit représenté un exemple de réalisation des moyens de liaison 6 permettent le clipsage du module de mesure 5 sur la cuillère 4. Cette caractéristique est particulièrement intéressante puisqu'elle permet de positionner le module de mesure 5 sur des forceps standards. Le clipsage permet également de retirer et de repositionner le module de mesure 5 de la cuillère céphalique 4 sans outil pour réaliser d'éventuelles opérations de remplacement de maintenance ou encore de lavage du module de mesure 5.

A cette fin comme représenté à la figure 2b les moyens de liaison 6 comportent une rainure 16 souple au niveau de chaque module de mesure 5. Cette rainure 16 permet l'insertion et la retenue des bords 17 de la cuillère céphalique 4.

En se reportant cette fois à la figure 3, on voit représenté un dispositif d'assistance 18, ce dispositif d'assistance 18 comprend un instrument obstétrical 1 et une unité de traitement 19.

L'unité de traitement 19 va permettre de calculer des données utiles pour le praticien.

L'unité de traitement 19 comporte ainsi des premiers moyens de traitement pour le calcul de la pression exercée et la comparaison avec au moins un seuil de pression maximale et/ou un seuil de pression maximale par unité de temps.

Selon un mode de réalisation avantageux, les premiers moyens de traitement comprennent un premier seuil S1 correspondant à une pression instantanée dans les normes et un second seuil S2 correspondant à une pression instantanée élevée et un troisième seuil correspondant à une pression instantanée critique.

Les moyens de traitement réalisent la comparaison entre soit la pression maximale exercée sur un capteur 10 parmi l'ensemble des capteurs soit une moyenne des pressions exercées sur les capteurs 10 et les valeurs de seuil S1, S2 et S3.

Le dispositif d'assistance 18 comprend en outre des moyens d'affichage 20 et/ou d'alarme correspondant aux résultats de l'unité de traitement. L'unité de traitement 19 transmet à ces moyens d'affichage 20 et/ou d'alarme les franchissements de seuil de pression instantanée ou de pression continue.

Selon un mode de réalisation simplifié, les premiers moyens de traitement comportent un seuil unique correspondant à une indication de pression instantanée trop élevée sur le fœtus.

Selon un autre mode de réalisation avantageux les premiers moyens de traitement comprennent un seuil Sc correspondant à un seuil de pression maximal admissible sur un temps donné.

En cas de dépassement du seuil Sc, une information spécifique sera affichée sur les moyens d'affichage 20 et/ou une alerte sera transmise par les moyens d'alarme.

Le dispositif d'assistance 18 comprend en outre des seconds moyens de traitement pour calculer un chemin d'extraction à partir des données des seconds moyens de mesure et comparer le chemin avec des données de positionnement de la base de données.

A cet effet le dispositif d'assistance 18 enregistre le déplacement du forceps à partir des coordonnés de positionnement du forceps sur le fœtus. La base de données comprend un chemin d'extraction type composé de deux segments de droites de longueur respective L1 et L2 formant entre elles un angle α. Les seconds moyens de traitement permettent de suivre le déplacement du forceps correspondant au chemin d'extraction du fœtus et de le comparer au chemin d'extraction type. Les seconds moyens de traitement permettent alors d'indiquer lorsque la longueur L1, à une marge près, est atteinte et indiquer si l'angle α entre les deux segments de droite, à une marge près est respecté.

L'unité de traitement comporte également de manière avantageuse des troisièmes moyens de traitement pour identifier à partir des premiers moyens de mesure la fréquence de contraction. Ces moyens de traitement permettent de réaliser une analyse des pressions mesurées et de retrouver dans les variations de pressions celles représentatives d'une contraction puis de stocker les informations temporelles relatives à ces contractions pour en déterminer la fréquence.

## Revendications

1. Instrument obstétrical notamment de type forceps pour l'extraction par traction d'un fœtus lors de l'accouchement comportant deux branches (2), chaque branche (2) étant équipée à une extrémité d'un manche de préhension (3) et à l'autre extrémité de cuillères céphaliques (4) **caractérisé en ce que** l'instrument comprend un module de mesure (5) par cuillère (4) et des moyens de liaison (6) entre le module de mesure (5) et la cuillère (4), ledit module de mesure (4) comportant :
- des premiers moyens de mesure (9) de la pression, aptes à mesurer la pression exercée par ladite cuillère (4) sur le fœtus,
- des seconds moyens de mesure (12) du positionnement dans l'espace de l'instrument obstétrical,
- des moyens d'alimentation électrique (14),
- des moyens de transmission (15) des mesures vers une unité de traitement.

2. Instrument obstétrical selon la revendication 1 dans lequel les moyens de liaison (6) permettent le clipsage du module de mesure (5) sur la cuillère céphalique (4).

3. Instrument obstétrical selon la revendication 2 dans lequel les moyens de liaison (6) comportent une rainure (16) souple au niveau de chaque module de mesure (5) permettant l'insertion et la retenue des bords (17) de la cuillère céphalique (4).

4. Instrument obstétrical selon l'une quelconque des revendications précédentes dans lequel les premiers moyens de mesure (9) de la pression comportent plusieurs capteurs (10) ponctuels repartis sur la face externe de chaque module de mesure.

5. Instrument obstétrical selon la revendication 3 dans lequel les premiers moyens de mesure de la pression comportent un capteur (10) reparti sur au moins 50% de la surface de la rainure (16).

6. Instrument obstétrical selon l'une quelconque des revendications précédentes dans lequel les premiers moyens de mesure (9) comportent au moins un capteur de type piézo-résistifs.

7. Instrument obstétrical selon l'une quelconque des revendications précédentes dans lequel les seconds moyens de mesure (12) comprennent une centrale inertielle (13).

8. Dispositif d'assistance comportant un instrument obstétrical selon l'une quelconque des revendications précédentes **caractérisé en ce qu'**il comprend en outre une unité de traitement (19) avec des premiers moyens de traitement pour le calcul de la pression exercée et la comparaison avec au moins un seuil de pression instantanée maximale et/ou un seuil de pression maximale par unité de temps.

9. Dispositif d'assistance selon la revendication 8 dans lequel l'unité de traitement (19) comprend des seconds moyens de traitement pour calculer un chemin d'extraction à partir des données des seconds moyens de mesure et comparer le chemin avec des données de positionnement de la base de données.

10. Dispositif d'assistance selon l'une ou l'autre des revendications 8 et 9 dans lequel l'unité de traitement (19) comporte des troisièmes moyens de traitement pour identifier à partir des premiers moyens de mesure la fréquence de contraction.

11. Dispositif d'assistance selon l'une quelconque des revendications 8 à 10 comportant des moyens d'affichage (20) et/ou d'alarme correspondant aux résultats de l'unité de traitement (19).

## Patentansprüche

1. Geburtshilfliches Instrument, insbesondere vom Typ einer Zange, zum Extrahieren eines Fötus durch Ziehen bei der Entbindung, das zwei Schenkel (2) umfasst, wobei jeder Schenkel (2) an einem Ende mit einem Griffstiel (3), und am anderen Ende mit Schädellöffeln (4) ausgestattet ist, **dadurch gekennzeichnet, dass** das Instrument ein Modul zum Messen (5) über den Löffel (4), und Mittel zur Verbindung (6) zwischen dem Messmodul (5) und dem Löffel (4) umfasst, wobei das Messmodul (4) umfasst:
- erste Mittel zum Messen (9) des Drucks, die dazu geeignet sind, den vom Löffel (4) auf den Fötus ausgeübten Druck zu messen,
- zweite Mittel zum Messen (12) der Lage des geburtshilflichen Instruments im Raum,
- Mittel zur Stromversorgung (14),
- Mittel zum Übertragen (15) der Messwerte an eine Verarbeitungseinheit.

2. Geburtshilfliches Instrument nach Anspruch 1, wobei die Verbindungsmittel (6) das Anklippen des Messmoduls (5) an den Schädellöffel (4) ermöglichen.

3. Geburtshilfliches Instrument nach Anspruch 2, wobei die Verbindungsmittel (6) im Bereich jedes Messmoduls (5) eine nachgiebige Nut (16) umfassen, die das Einführen und das Festhalten der Ränder (17) des Schädellöffels (4) ermöglicht.

4. Geburtshilfliches Instrument nach einem der vorstehenden Ansprüche, wobei die ersten Mittel zum Messen (9) des Drucks mehrere Punktsensoren (10) umfassen, die auf der Außenseite jedes Messmoduls verteilt sind.

5. Geburtshilfliches Instrument nach Anspruch 3, wobei die ersten Mittel zum Messen des Drucks einen Sensor (10) umfassen, der auf mindestens 50 % der Oberfläche der Nut (16) verteilt ist.

6. Geburtshilfliches Instrument nach einem der vorstehenden Ansprüche, wobei die ersten Messmittel (9) mindestens einen Sensor vom piezoresistiven Typ umfassen.

7. Geburtshilfliches Instrument nach einem der vorstehenden Ansprüche, wobei die zweiten Messmittel (12) eine inertiale Messeinheit (13) umfassen.

8. Hilfsvorrichtung, die ein geburtshilfliches Instrument nach einem der vorstehenden Ansprüche umfasst, **dadurch gekennzeichnet, dass** sie weiter eine Verarbeitungseinheit (19) mit ersten Verarbeitungsmitteln zur Berechnung des ausgeübten Drucks und den Vergleich mit mindestens einer Schwelle des maximalen Momentandrucks und/oder einer Schwelle des maximalen Drucks pro Zeiteinheit umfasst.

9. Hilfsvorrichtung nach Anspruch 8, wobei die Verarbeitungseinheit (19) zweite Verarbeitungsmittel zum Berechnen eines Extraktionsweges auf Grundlage der Daten der zweiten Messmittel, und Vergleichen des Weges mit Lagedaten aus der Datenbank umfasst.

10. Hilfsvorrichtung nach dem einen oder den anderen der Ansprüche 8 und 9, wobei die Verarbeitungseinheit (19) dritte Verarbeitungsmittel umfasst, um auf Grundlage der ersten Messmittel die Wehenfrequenz zu ermitteln.

11. Hilfsvorrichtung nach einem der Ansprüche 8 bis 10, die Mittel zur Anzeige (20) und/oder zur Warnung entsprechend den Ergebnissen der Verarbeitungseinheit (19) umfasst.

## Claims

1. An obstetrical instrument, in particular of the forceps type, for extracting by pulling a foetus during childbirth including two branches (2), each branch (2) being equipped, at one end, with a gripping handle (3) and, at the other end, with cephalic blades (4) **characterised in that** the instrument comprises one measurement module (5) per blade (4) and means (6) for connection between the measurement module (5) and the blade (4), said measurement module (4) including:
- first pressure measurement means (9), capable of measuring the pressure exerted by said blade (4) on the foetus,
- second means (12) for measuring the positioning of the obstetrical instrument in space,
- power supply means (14),
- means (15) for transmitting the measurements to a processing unit.

2. The obstetrical instrument according to claim 1, wherein the connection means (6) allow the measurement module (5) to be clipped onto the cephalic blade (4).

3. The obstetrical instrument according to claim 2, wherein the connection means (6) include a flexible groove (16) at each measurement module (5) allowing the insertion and retention of the edges (17) of the cephalic blade (4).

4. The obstetrical instrument according to any one of the preceding claims, wherein the first pressure measurement means (9) include several point sensors (10) distributed over the external face of each measurement module.

5. The obstetrical instrument according to claim 3, wherein the first pressure measurement means include a sensor (10) distributed over at least 50% of the surface of the groove (16) .

6. The obstetrical instrument according to any one of the preceding claims, wherein the first measurement means (9) include at least one piezo-resistive type sensor.

7. The obstetrical instrument according to any one of the preceding claims, wherein the second measurement means (12) comprise an inertial platform (13).

8. An assistance device including an obstetrical instrument according to any one of the preceding claims, **characterised in that** it further comprises a processing unit (19) with first processing means for calculating the exerted pressure and comparing it with at least one maximum instantaneous pressure threshold and/or one maximum pressure threshold per unit of time.

9. The assistance device according to claim 8, wherein the processing unit (19) comprises second processing means for calculating an extraction path from the data of the second measurement means and comparing the path with positioning data from the database.

10. An assistance device according to either of claims 8 and 9, wherein the processing unit (19) includes third processing means for identifying, from the first measurement means, the frequency of contraction.

11. The assistance device according to any one of claims 8 to 10, including display and/or alarm means (20) corresponding to the results of the processing unit (19).
